# EUROPEAN PATENT SPECIFICATION

(11) **EP 2 034 832 B1**
(45) Date of publication and mention of the grant of the patent: **02.10.2013**
(21) Application number: 07812248.8
(22) Date of filing: 22.06.2007
(51) Int. Cl.: C07D 241/04, A61K 31/495, A61P 11/00, A61P 11/06

(54) **IL-8 RECEPTOR ANTAGONIST**
IL-8-REZEPTOR-ANTAGONIST
ANTAGONISTE DU RÉCEPTEUR IL-8

(30) Priority: 23.06.2006 US 805626 P
(43) Date of publication of application: 18.03.2009
(73) Proprietor: GlaxoSmithKline LLC, Philadelphia, PA 19102 (US)
(72) Inventor: BUSCH-PETERSEN, Jakob, King Of Prussia, PA 19406 (US); BROOK, Christopher, S., King Of Prussia, PA 19406 (US); GOODMAN, Richard, M., Kink Of Prussia, PA 19406 (US); WEBB, Edward, C., King Of Prussia, PA 19406 (US)
(74) Representative: Povey, Alexander W.G.
(86) International application number: PCT/US2007/071866
(87) International publication number: WO 2007/150016

(56) References cited:
- US-A1- 2006 040 952
- US-A1- 2006 040 952

## Description

### FIELD OF THE INVENTION

This invention relates to N-[4-chloro-2-hydroxy-3-(piperazine-1-sulfonyl)phenyl]-N'-(2-chloro-3-fluorophenyl)urea p-toluenesulfonate, pharmaceutical compositions containing this compound, and its use in treating IL-8, GROα, GROβ; GROγ, NAP-2, and ENA-78 mediated diseases.

### BACKGROUND OF THE INVENTION

Many different names have been applied to Interleukin-8 (IL-8), such as neutrophil attractant/activation protein-1 (NAP-1), monocyte derived neutrophil chemotactic factor (MDNCF), neutrophil activating factor (NAF), and T-cell lymphocyte chemotactic factor. Interleukin-8 is a chemoattractant for neutrophils, basophils, and a subset of T-cells. It is produced by a majority of nucleated cells including macrophages, fibroblasts, endothelial and epithelial cells exposed to TNF, IL-1α, IL-1β or LPS, and by neutrophils themselves when exposed to LPS or chemotactic factors such as FMLP.

GROα, GROβ, GROγ and NAP-2 also belong to the chemokine α family. Like IL-8, these chemokines have also been referred to by different names. For instance GROα, β, γ have been referred to as MGSAα, β and γ respectively (Melanoma Growth Stimulating Activity). All of the chemokines of the α-family which possess the ELR motif directly preceding the CXC motif bind to the IL-8 B receptor (CXCR2).

IL-8, GROα, GROβ, GROγ, NAP-2, and ENA-78 stimulate a number of functions in vitro. They have all been shown to have chemoattractant properties for neutrophils, while IL-8 and GROα have demonstrated T-lymphocyte and basophilic chemotactic activity. In addition, IL-8 can induce histamine release from basophils from both normal and atopic individuals. GRO-α and IL-8 can, in addition, induce lysozomal enzyme release and respiratory burst from neutrophils. IL-8 has also been shown to increase the surface expression of Mac-1 (CD11b/CD18) on neutrophils without de novo protein synthesis. This may contribute to increased adhesion of the neutrophils to vascular endothelial cells. Many known diseases are characterized by massive neutrophil infiltration. As IL-8, GROα, GROβ, GROγ and NAP-2 promote the accumulation and activation of neutrophils, these chemokines have been implicated in a wide range of acute and chronic inflammatory disorders including psoriasis and rheumatoid arthritis. In addition the ELR chemokines (those containing the amino acids ELR motif just prior to the CXC motif) have also been implicated in angiostasis.

In vitro, IL-8, GROα, GROβ, GROγ and NAP-2 induce neutrophil shape change, chemotaxis, granule release, and respiratory burst, by binding to and activating receptors of the seven-transmembrane, G-protein-linked family, in particular by binding to IL-8 receptors, most notably the IL-8β receptor (CXCR2). The development of non-peptide small molecule antagonists for members of this receptor family has precedent. Hence, the 1L-8 receptor represents a promising target for the development of novel anti-inflammatory agents.

There remains a need for treatment, in this field, for compounds, which are capable of binding to the CXCR 1 and/or CXCR2 receptors. Therefore, conditions associated with an increase in IL-8 production (which is responsible for chemotaxis of neutrophil and T-cells subsets into the inflammatory site) would benefit by compounds which are inhibitors of IL-8 receptor binding. Such compounds are disclosed in WO 2004/039775, U.S. patent number 6,180,675 and U.S. patent number 6,500,863.

### SUMMARY OF THE INVENTION

The present invention relates to N-[4-chloro-2-hydroxy-3-(piperazine-1-sulfonyl)phenyl]-N'-(2-chloro-3-fluorophenyl)urea p-toluenesulfonate (present compound), and compositions comprising the present compound and a pharmaceutically acceptable carrier or diluent.

The present invention also relates to combinations comprising the present compound and one or more additional therapeutic ingredients.

Also described is a method of treating a chemokine mediated disease wherein the chemokine is one which binds to an IL-8α or β receptor, and which method comprises administering an effective amount of the present compounds.

Also described is method of inhibiting the binding of IL-8 to its receptors in a mammal, particularly in a human, in need thereof which comprises administering an effective amount of the present compound.

### DETAILED DESCRIPTION OF THE INVENTION

The present invention relates to N-[4-chloro-2-hydroxy-3-(piperazine-1-sulfonyl)phenyl]-N'-(2-chloro-3-fluorophenyl)urea p-toluenesulfonate and compositions comprising the present compound and a pharmaceutically acceptable carrier or diluent.

The present invention also relates to combinations comprising the present compound and one or more additional therapeutic ingredients.

Also described is a method of treating a chemokine mediated disease wherein the chemokine is one which binds to an IL-8α or β receptor, and which method comprises administering an effective amount of the present compound.

Also described is a method of inhibiting the binding of IL-8 to its receptors in a mammal, particularly in a human, in need thereof which comprises administering an effective amount of the present compound.

Also described are the methods of treating asthma, chronic obstructive pulmonary disease and adult respiratory disease. In particular, the instant invention relates to treating obstructive pulmonary disease using the present compound.

### METHOD OF PREPARATION

### N-[4-chloro-2-hydroxy-3-(piperazine-1-sulfonyl)phenyl]-N'-(2-chloro-3-fluorophenyl)urea p-toluenesulfonate

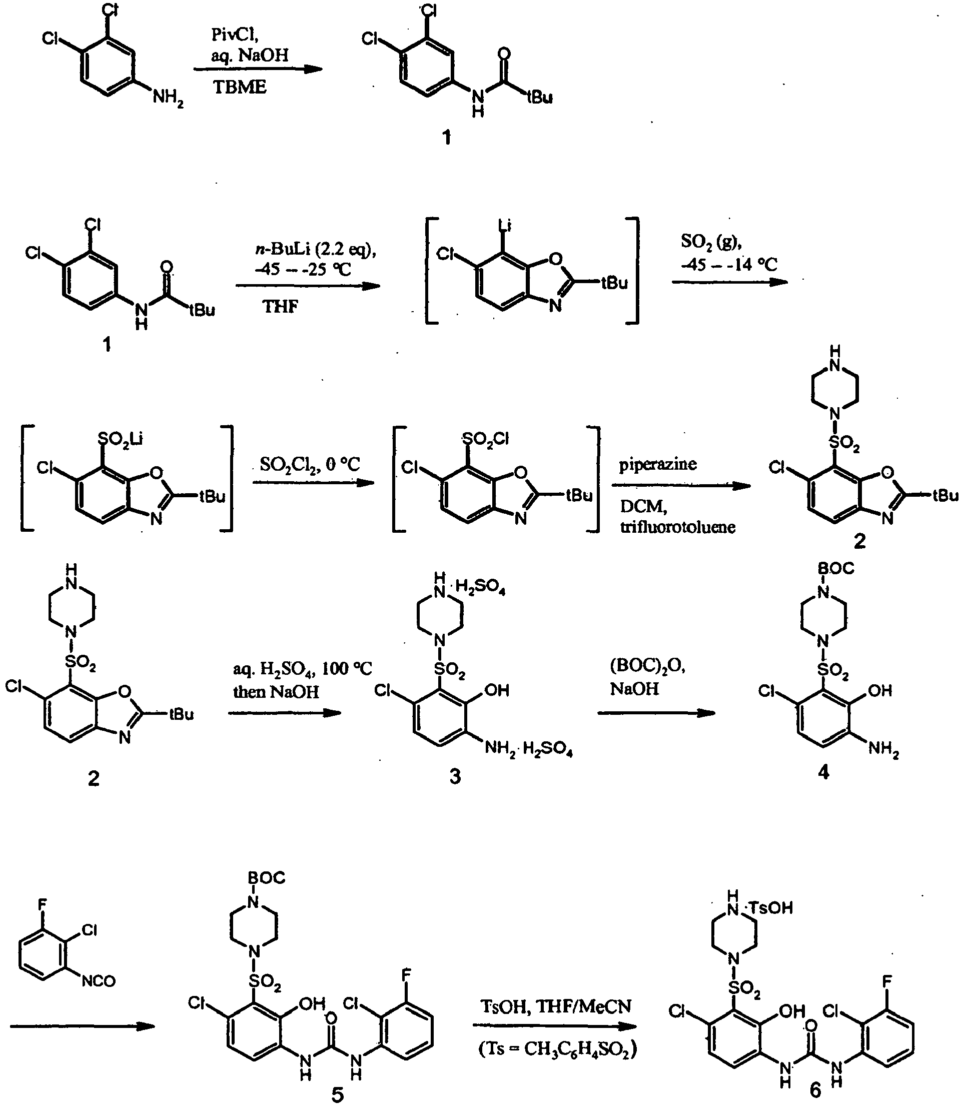

### Preparation of Compound 1

3,4-dichloroaniline (100 g) was dissolved in tetrabutylmethylamine (TBME) (660 mL) and cooled to 10-15 °C. Sodium hydroxide (94 g of a 30% aqueous solution) was added, and the solution stirred vigorously via mechanical stirrer. Trimethylacetyl chloride (84 mL) was added at such a rate as to keep the internal temperature below 35 °C. When the addition was complete (10-15 min), the mixture was maintained at 30-35 °C for about 30 min, and then cooled to 0-5 °C over 30-40 minutes. The reaction mixture was held at 0-5 °C for 1hr, and then filtered, rinsing first with 90:10 water/methanol (400 mL) and then water (600 mL.) Drying at 50-55 °C under vacuum afforded product as off-white crystals. A yield of 127 g was obtained.

### Preparation of Compound 2

A solution of Compound 1 (300 mL) was cooled to -50 - -40 °C under an inert atmosphere of nitrogen. N-Butyl lithium (2.5M in hexanes, 179 mL) was added at such a rate as to keep the solution's internal temperature between -45 - -30 °C (ca. 15 - 30 min addition). The solution was held at ca. -35 - -25 °C until HPLC indicated that the initial reaction was complete. The solution was then recooled to -45 - -40 °C, and sulfur dioxide (∼16.9 g) was bubbled through the solution, keeping the internal temperature below approximately -14 °C, until the solution was acidic. When the reaction was complete, the mixture was warmed to -10 - 0 °C. Starting at -2 - 3 °C, sulfuryl chloride (25.2 mL) was then added dropwise to the tetrahydrofuran solution over 5 - 15 min, keeping the temperature below approximately 22 °C. After 5 min, HPLC confirmed reaction completion, while the solution was kept around 10 - 15 °C. The mixture was solvent-exchanged into α,α,α-trifluorotoluene under reduced pressure, filtered, partially concentrated under vacuum (to ∼100 mL), followed by addition of dichloromethane (350 mL). To this mixture was added a solution of piperazine (61.2 g) in dichloromethane (625 mL) at ambient temperature dropwise, keeping the solution's internal temperature at 15 - 27 °C (2h addition). The reaction was held at 20 - 24 °C until complete. The mixture was washed with deionized water (200 mL), the organic layer concentrated, followed by addition of heptane (450 mL). The product (70.5 g) was isolated by filtration, washed with heptane (50-100 mL), and dried under vacuum at 50-55 °C.

### Preparation of Compound 3

Compound 2 (30 g) was added to ∼16% (w/w in water) sulfuric acid (300 mL). The resulting mixture was heated to reflux at 99-103°C for ∼6 hours. Upon completion of the reaction, the solution was cooled to 40-50 °C, then concentrated to ∼60 mL under reduced pressure. Acetonitrile (225 mL) was added and the resulting suspension stirred at 20-25 °C for ∼1 hour. The product was isolated by filtration, washed with acetonitrile (135 mL) and dried at 45-50 °C under vacuum. A yield of 33.34 g was obtained.

### Preparation of Compound 4

Compound 3 (20 g) was added to deionized water (200 mL). The pH of the resulting solution was adjusted to 6.5 - 7.0 by adding 50% aq. sodium hydroxide (∼6.35 mL) while maintaining the internal temperature between 20 - 30 °C. Then a solution of di-tert-butyl dicarbonate (8.9 g) in ethyl acetate (80 mL + 20 mL rinse) was added. The pH of the resulting mixture was adjusted to 6.8 - 7.0 by adding 50% aq. sodium hydroxide (2.45 mL) while maintaining the internal temperature between 20 - 30 °C. Upon completion of the reaction, the reaction solution is filtered to remove the small amount of precipitate. The two layers of the filtrate were separated, and the aqueous layer was extracted with ethyl acetate (140 mL). Combined ethyl acetate layers are washed with water (40 mL) and concentrated to 100 mL. Heptane (100 mL) was added and the resulting suspension was concentrated to 60 mL. This process was repeated once more. Heptane (140 mL) was then added, and the resulting suspension was stirred at 20 - 25 °C for ∼1 hour. The product was isolated by filtration, washed with heptane (80 mL) and dried at 40 - 45 °C under vacuum. A yield of 15.3 g was obtained.

### Preparation of Compound 5

Compound 4 (10 g) was added to dimethylformamide (20 mL) and acetonitrile (80 mL). 2-Chloro-3-fluorophenyl isocyanate (4.77 g) was added while maintaining the internal temperature between 20-30 °C, followed by 10 mL acetonitrile rinse. The resulting mixture was stirred at 20-25 °C for ∼2 hours. Upon completion of the reaction, methanol (50 mL) was added. The resulting suspension was stirred at 20-25 °C for ∼10 minutes. Deionized water (150 mL) was added, and the resulting suspension stirred at 2025 °C for ∼1 hour. The product was isolated by filtration, washed with deionized water (100 mL) and methanol (15-20 mL), and then dried at 40-45 °C under vacuum. A yield of 14.15g was obtained.

### Preparation of Compound 6 - Procedure 1

Compound 5 (50 g) was dissolved in tetrahydrofuran (THF, 200 mL) and heated to 33-37 °C and held at 33-37 °C. In another reactor, a solution of acetonitrile (250 mL), THF (50 mL) and p-toluenesulfonic acid monohydrate (43.9 g) was prepared. The resulting solution was heated to 33-37 °C and held at 33-37 °C. The p-toluenesulfonic acid solution was filtered and transferred into the reactor containing Compound 5 and THF while maintaining the temperature at 33-37 °C. After the starting material was consumed, micronized seeds of product (0.5 g) were charged in a minimal amount of acetonitrile (5 mL). The reaction mixture was then heated to 53-57 °C over ∼40 minutes, and held at that temperature for at least 4 hours. The reaction was cooled to 0-5 °C, the product isolated by filtration, washed with acetonitrile (250 mL), and dried under vacuum at 55-60 °C. A yield of 52.24g was obtained.

### Preparation of Compound 6 - Procedure 2

Compound 5 (500 g) was charged to reactor 1 followed by acetonitrile (CAN, 3750 mL) and tetrahydrofuran (THF, 1250 mL). The solution was then heated to 60-65 °C and once a clear solution is observed, a clarifying filtration is performed to reactor 2. To reactor 1, p-toluenesulfonic acid monohydrate (TsOH·H₂O, 439 g) is added followed by ACN (750 mL) and THF (250 mL). The mixture was heated to 40-45 °C and once a clear solution was observed, a clarifying filtration was performed, adding the solution to reactor 2 (containing the starting material solution) and maintaining the temperature in reactor 2 at 50-60 °C. The mixture was heated to reflux, and held at 70-80 °C until the reaction was complete. ∼3500 mL of solvent was removed by atmospheric distillation. The reactor was then charged with 2.5 L water followed by 4 L ACN, and the temperature adjusted to 70-80 °C. After dissolution was observed, the resulting solution was cooled to 64-68 °C. After 5-10 minutes, milled product seeds (5 g) were added in a minimal amount of acetonitrile, and held at 64-68 °C for one hour. The mixture was cooled to 0-5 °C over 2 hours and held at 0-5 °C for ∼30 minutes before isolating the product by filtration. The solid product was washed with 2.5L of acetonitrile, and dried under vacuum at 50-60 °C. A yield of 480 g was obtained.

The present compound is useful in the manufacture of a medicine for the prophylactic or therapeutic treatment of any disease state in a human, or other mammal, which is exacerbated or caused by excessive or unregulated IL-8 cytokine production by such mammal's cell, such as, but not limited to, monocytes and/or macrophages, or other chemokines which bind to the IL-8 α or β receptor, also referred to as the type I or type II receptor.

Also described is a method of treating a chemokine mediated disease, wherein the chemokine is one which binds to an IL-8 α or β receptor and which method comprises administering an effective amount of the present compound. In particular, the chemokines are IL-8, GROα, GROβ, GROγ, NAP-2 or ENA-78.

The present compound is administered in an amount sufficient to inhibit cytokine function, in particular IL-8, GROα, GROβ, GROγ, NAP-2 or ENA-78, such that they are biologically regulated down to normal levels of physiological function, or in some case to subnormal levels, so as to ameliorate the disease state. Abnormal levels of IL-8, GROα, GROβ, GROγ, NAP-2 or ENA-78, for instance, in the context of the present invention, constitute: (i) levels of free IL-8 greater than or equal to 1 picogram per mL; (ii) any cell associated IL-8, GROα, GROβ, GROγ, NAP-2 or ENA-78 above normal physiological levels; or (iii) the presence of IL-8, GROα, GROβ, GROγ, NAP-2 or ENA-78 above basal levels in cells or tissues in which IL-8, GROα, GROβ, GROγ, NAP-2 or ENA-78 respectively, is produced.

There are many disease states in which excessive or unregulated IL-8 production is implicated in exacerbating and/or causing the disease. Chemokine mediated diseases include psoriasis, atopic dermatitis, osteo arthritis, rheumatoid arthritis, asthma, chronic obstructive pulmonary disease, adult respiratory distress syndrome, inflammatory bowel disease, Crohn's disease, ulcerative colitis, stroke, septic shock, endotoxic shock, gram negative sepsis, toxic shock syndrome, cardiac and renal reperfusion injury, glomerulonephritis, thrombosis, graft vs. host reaction, alzheimers disease, allograft rejections, malaria, restinosis, angiogenesis, atherosclerosis, osteoporosis, gingivitis, viral diseases such as rhinovirus or undesired hematopoietic stem cell release.

In particular, the compound of the present invention is useful in the treatment of asthma, chronic obstructive pulmonary disease and adult respiratory distress syndrome. Preferably, the present compound is useful for treating chronic obstructive pulmonary disease.

The diseases of the present invention are primarily characterized by massive neutrophil infiltration, T-cell infiltration, or neovascular growth, and are associated with increased IL-8, GROα, GROβ, GROγ, NAP-2 or ENA-78 production which is responsible for the chemotaxis of neutrophils into the inflammatory site or the directional growth of endothelial cells. In contrast to other inflammatory cytokines (IL-1, TNF, and IL-6), IL-8, GROα, GROβ, GROγ, NAP-2 or ENA-78 have the unique property of promoting neutrophil chemotaxis, enzyme release including but not limited to elastase release as well as superoxide production and activation. The α-chemokines, but particularly, GROα, GROβ, GROγ, NAP-2 or ENA-78, working through the IL-8 type I or II receptor, can promote the neovascularization of tumors by promoting the directional growth of endothelial cells. Therefore, the inhibition of IL-8 induced chemotaxis or activation would lead to a direct reduction in the neutrophil infiltration.

Recent evidence also implicates the role of chemokines in the treatment of HIV infections, Littleman et al., Nature 381, pp. 661 (1996) and Koup et al., Nature 381, pp. 667 (1996).

Present evidence also indicates the use of IL-8 inhibitors in the treatment of atherosclerosis. The first reference, Boisvert et al., J. Clin. Invest, 1998, 101:353-363 shows, through bone marrow transplantation, that the absence of IL-8 receptors on stem cells (and, therefore, on monocytes/macrophages) leads to a reduction in the development of atherosclerotic plaques in LDL receptor deficient mice.

The present invention also provides for a means of treating CNS injuries. Such treatment is provided in an acute setting, as well as for prevention of injury in those individuals deemed susceptible to injury.

CNS injuries as defined herein include both open or penetrating head trauma, such as by surgery, or a closed head trauma injury, such as by an injury to the head region. Also included within this definition is ischemic stroke, particularly to the brain area.

Ischemic stroke may be defined as a focal neurologic disorder that results from insufficient blood supply to a particular brain area, usually as a consequence of an embolus, thrombi, or local atheromatous closure of the blood vessel. The role of inflammatory cytokines in this area has been emerging and the present invention provides means for the potential treatment of these injuries. Relatively little treatment, for an acute injury such as these has been available.

TNF-α is a cytokine with proinflammatory actions, including endothelial leukocyte adhesion molecule expression. Leukocytes infiltrate into ischemic brain lesions and hence compounds which inhibit or decrease levels of TNF would be useful for treatment of ischemic brain injury. See Liu et al., Stroke, Vol. 25., No. 7, pp. 1481-88 (1994).

Models of closed head injuries and treatment with mixed 5-LO/CO agents is discussed in Shohami et al., J. of Vaisc & Clinical Physiology and Pharmacology, Vol. 3, No. 2, pp. 99-107 (1992). Treatment which reduced edema formation was found to improve functional outcome in those animals treated.

The present compound is administered in an amount sufficient to inhibit IL-8, binding to the IL-8 alpha or beta receptors, from binding to these receptors, such as evidenced by a reduction in neutrophil chemotaxis and activation. The discovery that the present compound is an inhibitor of IL-8 binding is based upon the effects of the present compound in the assays.

As used herein, the term "IL-8 mediated disease or disease state" refers to any and all disease states in which IL-8, GROα, GROβ, GROγ, NAP-2 or ENA-78 plays a role, either by production of IL-8, GROα, GROβ, GROγ, NAP-2 or ENA-78 themselves, or by IL-8, GROα, GROβ, GROγ, NAP-2 or ENA-78 causing another monokine to be released, such as but not limited to IL-1, IL-6 or TNF. A disease state in which, for instance, IL-1 is a major component, and whose production or action, is exacerbated or secreted in response to IL-8, would therefore be considered a disease state mediated by IL-8.

As used herein, the term "chemokine mediated disease or disease state" refers to any and all disease states in which a chemokine which binds to an IL-8α or β receptor plays a role, such as but not limited to IL-8, GRO-α, GRO-β, GROγ, NAP-2 or ENA-78. This would include a disease state in which, IL-8 plays a role, either by production of IL-8 itself, or by IL-8 causing another monokine to be released, such as but not limited to IL-1, IL-6 or TNF. A disease state in which, for instance, IL-1 is a major component, and whose production or action, is exacerbated or secreted in response to IL-8, would therefore be considered a disease stated mediated by IL-8.

As used herein, the term "cytokine" refers to any secreted polypeptide that affects the functions of cells and is a molecule which modulates interactions between cells in the immune, inflammatory or hematopoietic response. A cytokine includes, but is not limited to, monokines and lymphokines, regardless of which cells produce them. For instance, a monokine is generally referred to as being produced and secreted by a mononuclear cell, such as a macrophage and/or monocyte. Many other cells however also produce monokines, such as natural killer cells, fibroblasts, basophils, neutrophils, endothelial cells, brain astrocytes, bone marrow stromal cells, epideral keratinocytes and B-lymphocytes. Lymphokines are generally referred to as being produced by lymphocyte cells. Examples of cytokines include, but are not limited to, Interleukin-1 (IL-1), Interleukin-6 (IL-6), Interleukin-8 (IL-8), Tumor Necrosis Factor-alpha (TNF-α) and Tumor Necrosis Factor beta (TNF-β).

As used herein, the term "chemokine" refers to any secreted polypeptide that affects the functions of cells and is a molecule which modulates interactions between cells in the immune, inflammatory or hematopoietic response, similar to the term "cytokine" above. A chemokine is primarily secreted through cell transmembranes and causes chemotaxis and activation of specific white blood cells and leukocytes, neutrophils, monocytes, macrophages, T-cells, B-cells, endothelial cells and smooth muscle cells. Examples of chemokines include, but are not limited to IL-8, GRO-α, GRO-β, GRO-γ, NAP-2, ENA-78, IP-10, MIP-1α, MIP-β, PF4, and MCP 1, 2, and 3.

In order to use the present compound in therapy, it will normally be formulated into a pharmaceutical composition in accordance with standard pharmaceutical practice. This invention, therefore, also relates to a pharmaceutical composition comprising an effective, non-toxic amount of the present compound and a pharmaceutically acceptable carrier or diluent.

The present compound and pharmaceutical compositions incorporating such may conveniently be administered by any of the routes conventionally used for drug administration, for instance, orally, topically, parenterally or by inhalation. The present compound may be administered in conventional dosage forms prepared by combining the present compound with standard pharmaceutical carriers according to conventional procedures. The present compound may also be administered in conventional dosages in combination with a known, second therapeutically active compound. These procedures may involve mixing, granulating and compressing or dissolving the ingredients as appropriate to the desired preparation. It will be appreciated that the form and character of the pharmaceutically acceptable character or diluent is dictated by the amount of active ingredient with which it is to be combined, the route of administration and other well-known variables. The carrier(s) must be "acceptable" in the sense of being compatible with the other ingredients of the formulation and not deleterious to the recipient thereof.

The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are syrup, peanut oil, olive oil, water and the.like. Similarly, the carrier or diluent may include time delay material well known to the art, such as glyceryl mono-stearate or glyceryl distearate alone or with a wax.

A wide variety of pharmaceutical forms can be employed. Thus, if a solid carrier is used, the preparation can be tableted, placed in a hard gelatin capsule in powder or pellet form or in the form of a troche or lozenge. The amount of solid carrier will vary widely but preferably will be from about 25mg to about 1g. When a liquid carrier is used, the preparation will be in the form of a syrup, emulsion, soft gelatin capsule, sterile injectable liquid such as an ampule or nonaqueous liquid suspension.

The present compound may be administered topically, that is by non-systemic administration. This includes the application of the present compound externally to the epidermis or the buccal cavity and the instillation of such a compound into the ear, eye and nose, such that the compound does not significantly enter the blood stream. In contrast, systemic administration refers to oral, intravenous, intraperitoneal and intramuscular administration.

Formulations suitable for topical administration include liquid or semi-liquid preparations suitable for penetration through the skin to the site of inflammation such as liniments, lotions, creams, ointments or pastes, and drops suitable for administration to the eye, ear or nose. The active ingredient may comprise, for topical administration, from 0.001% to 10% w/w, for instance from 1% to 2% by weight of the formulation. It may however comprise as much as 10% w/w but preferably will comprise less than 5% w/w, more preferably from 0.1% to 1% w/w of the formulation.

Lotions according to the present invention include those suitable for application to the skin or eye. An eye lotion may comprise a sterile aqueous solution optionally containing a bactericide and may be prepared by methods similar to those for the preparation of drops. Lotions or liniments for application to the skin may also include an agent to hasten drying and to cool the skin, such as an alcohol or acetone, and/or a moisturizer such as glycerol or an oil such as castor oil or arachis oil.

Creams, ointments or pastes according to the present invention are semi-solid formulations of the active ingredient for external application. They may be made by mixing the active ingredient in finely-divided or powdered form, alone or in solution or suspension in an aqueous or non-aqueous fluid, with the aid of suitable machinery, with a greasy or non-greasy base. The base may comprise hydrocarbons such as hard, soft or liquid paraffin, glycerol, beeswax, a metallic soap; a mucilage; an oil of natural origin such as almond, corn, arachis, castor or olive oil; wool fat or its derivatives or a fatty acid such as steric or oleic acid together with an alcohol such as propylene glycol or a macrogel. The formulation may incorporate any suitable surface active agent such as an anionic, cationic or non-ionic surfactant such as a sorbitan ester or a polyoxyethylene derivative thereof. Suspending agents such as natural gums, cellulose derivatives or inorganic materials such as silicaceous silicas, and other ingredients such as lanolin, may also be included.

Drops according to the present invention may comprise sterile aqueous or oily solutions or suspensions and may be prepared by dissolving the active ingredient in a suitable aqueous solution of a bactericidal and/or fungicidal agent and/or any other suitable preservative, and preferably including a surface active agent. The resulting solution may then be clarified by filtration, transferred to a suitable container which is then sealed and sterilized by autoclaving or maintaining at 98-100°C for half an hour. Alternatively, the solution may be sterilized by filtration and transferred to the container by an aseptic technique. Examples of bactericidal and fungicidal agents suitable for inclusion in the drops are phenylmercuric nitrate or acetate (0.002%), benzalkonium chloride (0.01%) and chlorhexidine acetate (0.01%). Suitable solvents for the preparation of an oily solution include glycerol, diluted alcohol and propylene glycol.

The present compound may be administered parenterally, that is by intravenous, intramuscular, subcutaneous, intranasal, intrarectal, intravaginal or intraperitoneal administration. Appropriate dosage forms for such administration may be prepared by conventional techniques. The present compound may also be administered by inhalation, that is by intranasal and oral inhalation administration. Appropriate dosage forms for such administration, such as an aerosol formulation or a metered dose inhaler, may be prepared by conventional techniques.

For all methods of use disclosed herein for the present compound, the daily oral dosage regimen will preferably be from about 0.01 to about 80 mg/kg of total body weight. The daily parenteral dosage regimen about 0.001 to about 80 mg/kg of total body weight. The daily topical dosage regimen will preferably be from 0.1 mg to 150 mg, administered one to four, preferably two or three times daily. The daily inhalation dosage regimen will preferably be from about 0.01 mg/kg to about 1 mg/kg per day. It will also be recognized by one of skill in the art that the optimal quantity and spacing of individual dosages of the present compound will be determined by the nature and extent of the condition being treated, the form, route and site of administration, and the particular patient being treated, and that such optimums can be determined by conventional techniques. It will also be appreciated by one of skill in the art that the optimal course of treatment, i.e., the number of doses of the present compound given per day for a defined number of days, can be ascertained by those skilled in the art using conventional course of treatment determination tests.

### Combinations:

The present compound and pharmaceutical formulations according to the invention may he used in combination with or include one or more other therapeutic agents, for example selected from anti-inflammatory agents, anticholinergic agents (particularly an M₁/M₂/M₃ receptor antagonist), β₂-adrenoreceptor agonists, antiinfective agents, such as antibiotics, antivirals, or antihistamines. The invention thus provides, in a further aspect, a combination comprising the present compound or physiologically functional derivatives of the present compound together with one or more other therapeutically active agents, for example selected from an anti-inflammatory agent, such as a corticosteroid or an NSAID, an anticholinergic agent, a β₂-adrenoreceptor agonist, an antiinfective agent, such as an antibiotic or an antiviral, or an antihistamine. One embodiment of the invention encompasses combinations comprising the present compound or physiologically functional derivative thereof together with a β₂-adrenoreceptor agonist, and/or an anticholinergic, and/or a PDE-4 inhibitor, and/or an antihistamine.

It will be clear to a person skilled in the art that, where appropriate, the other therapeutic ingredient(s) may be used in the form of salts, for example as alkali metal or amine salts or as acid addition salts, or prodrugs, or as esters, for example lower alkyl esters, or as solvates, for example hydrates to optimise the activity and/or stability and/or physical characteristics, such as solubility, of the therapeutic ingredient. It will be clear also that, where appropriate, the therapeutic ingredients may be used in optically pure form.

In one embodiment, the invention encompasses a combination comprising the present compound together with a β₂-adrenoreceptor agonist. Examples of β₂-adrenoreceptor agonists include salmeterol (which may be a racemate or a single enantiomer such as the *R*-enantiomer), salbutamol (which may be a racemate or a single enantiomer such as the *R*-enantiomer), formoterol (which may be a racemate or a single diastereomer such as the *R,R*-diastereomer), salmefamol, fenoterol, carmoterol, etanterol, naminterol, clenbuterol, pirbuterol, flerbuterol, reproterol, bambuterol, indacaterol, terbutaline and salts thereof, for example the xinafoate (1-hydroxy-2-naphthalenecarboxylate) salt of salmeterol, the sulphate salt or free base of salbutamol or the fumarate salt of formoterol. In one embodiment the β₂-adrenoreceptor agonists are long-acting B₂-adrenoreceptor agonists, for example, compounds which provide effective bronchodilation for about 12 hours or longer. Other β₂-adrenoreceptor agonists include those described in WO2002/066422, WO2002/070490, WO2002/076933, WO2003/024439, WO2003/072539, WO2003/091204, WO2004/016578, WO2004/022547, WO2004/037807, WO2004/037773, WO2004/037768, WO2004/039762, WO2004/039766, WO02001/42193 and WO2003/042160.

Further examples of β₂-adrenoreceptor agonists include:
3-(4- {[6-({(2 R)-2-hydroxy-2-[4-hydroxy-3-(hydroxymethyl)phenyl]ethyl} amino) hexyl] oxy} butyl) benzenesulfonamide;
3-(3-{[7-({(2R)-2-hydroxy-2-[4-hydroxy-3-hydroxymethyl) phenyl] ethyl}-amino) heptyl] oxy} propyl) benzenesulfonamide;
4-{(1R)-2-[(6-{2-[(2, 6-dichlorobenzyl)oxy]ethoxy} hexyl) amino]-1-hydroxyethyl}-2-(hydroxymethyl) phenol;
4-{(1R)-2-[(6-{4-[3-(cyclopentylsulfonyl)phenyl]butoxy}hexyl)amino]-1-hydroxyethyl}-2-(hydroxymethyl)phenol;
N-[2-hydroxyl-5-[(1R)-1-hydroxy-2-[[2-4-[[(2R)-2-hydroxy-2-phenylethyl]amino]phenyl]ethyl]amino]ethyl]phenyl]formamide;
N-2{2-[4-(3-phenyl-4-methoxyphenyl)aminophenyl]ethyl}-2-hydroxy-2-(8-hydroxy-2(1H)-quinolinon-5-yl)ethylamine; and
5-[(R)-2-(2- {4-[4-(2-amino-2-methyl-propoxy)-phenylamino] -phenyl}-ethylamino)-1-hydroxy-ethy]-8-hydroxy-1H-quinolin-2-one.

The β₂-adrenoreceptor agonist may be in the form of a salt formed with a pharmaceutically acceptable acid selected from sulphuric, hydrochloric, fumaric, hydroxynaphthoic (for example 1- or 3-hydroxy-2-naphthoic), cinnamic, substituted cinnamic, triphenylacetic, sulphamic, sulphanilic, naphthaleneacrylic, benzoic, 4-methoxybenzoic, 2- or 4-hydroxybenzoic, 4-chlorobenzoic and 4-phenylbenzoic acid.

Suitable anti-inflammatory agents include corticosteroids. Examples of corticosteroids which may be used in combination with the compounds of the invention are those oral and inhaled corticosteroids and their pro-drugs which have anti-inflammatory activity.

Examples include methyl prednisolone, prednisolone, dexamethasone, fluticasone propionate, 6α,9α-difluoro-11β-hydroxy-16α-methyl-17α-[(4-methyl-1,3-thiazole-5-carbonyl)oxyl-3-oxo-androsta-1,4-diene-17β-carbothioic acid S-fluoromethyl ester, 6α,9α-difluoro-17α-[(2-furanylcarbonyl)oxy]-11β-hydroxy-16α-methyl-3-oxo-androsta-1,4-diene-17β-carbothioic acid S-fluoromethyl ester (fluticasone furoate), 6α,9α-difluoro-11β-hydroxy-16α-methyl-3-oxo-17α-propionyloxy- androsta-1,4-diene-17β-carbothioic acid S-(2-oxo-tetrahydro-furan-3S-yl) ester, 6α,9α-difluoro-11β-hydroxy-16α-methyl-3-oxo-17α-(2,2,3,3- tetramethycyclopropylcarbonyl)oxy-androsta-1,4-diene-17β-carbothioic acid S-cyanomethyl ester and 6α,9α-difluoro-11β-hydroxy-16α-methyl-17α-(1-methycyclopropylcarbonyl)oxy-3-oxo-androsta-1,4-diene-17β-carbothioic acid S-fluoromethyl ester, beclomethasone esters (for example the 17-propionate ester or the 17,21-dipropionate ester), budesonide, flunisolide, mometasone esters (for example mometasone furoate), triamcinolone acetonide, rofleponide, ciclesonide (16□,17-[[(R)-cyclohexylmethylene]bis(oxy)]-11□,21-dihydroxy-pregna-1,4-diene-3,20-dione), butixocort propionate, RPR-106541, and ST-126. In one embodiment corticosteroids include fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester, 6α,9α-difluoro-17α-[(2-furanylcarbonyl)oxy]-11β-hydroxy-16α-methyl-3-oxo-androsta-1,4-diene-17β-carbothioic acid S-fluoromethyl ester, 6α,9α-difluoro-11β-hydroxy-16α-methyl-3-oxo-17α-(2,2,3,3- tetramethycyclopropylcarbonyl)oxy-androsta-1,4-diene-17β-carbothioic acid S-cyanomethyl ester and 6α,9α-difluoro-11β-hydroxy-16α-methyl-17α-(1-methycyclopropylcarbonyl)oxy-3-oxo-androsta-1,4-,diene-17β-carbothioic acid S-fluoromethyl ester. In one embodiment the corticosteroid is 6α,9α-difluoro-17α-[(2-furanylcarbonyl)oxy]-11β-hydroxy-16α-methyl-3-oxo-androsta-1,4-diene-17β-carbothioic acid S-fluoromethyl ester.

Examples of corticosteroids also include those described in WO2002/088167, WO2002/100879, WO2002/12265, WO2002/12266, WO2005/005451, WO2005/005452, WO2006/072599 and WO2006/072600.

Non-steroidal compounds having glucocorticoid agonism that may possess selectivity for transrepression over transactivation and that may be useful in combination therapy include those covered in the following published patent Applications and patents: WO2003/082827, WO1998/54159, WO2004/005229, WO2004/009017, WO2004/018429, WO2003/104195, WO2003/082787, WO2003/082280, WO2003/059899, WO2003/101932 WO2002/02565, WO2001/16128, WO2000/66590, WO2003/086294, WO2004/026248 WO2003/061651 W02003/08277, W02006/000401, WO2006/000398 and WO2006/015870.

Non-steroidal compounds having glucocorticoid agonism that may possess selectivity for transrepression over transactivation and that may be useful in combination therapy include those covered in the following patents: WO2003/082827, WO1998/54159, WO2004/005229, WO2004/009017, WO2004/018429, WO2003/104195, W02003/082787, WO2003/082280, W02003/059899, WO2003/101932, WO2002/02565, WO2001/16128, W02000/66590, W02003/086294, WO2004/026248, WO2003/061651 and W02003/08277.

Examples of anti-inflammatory agents include non-steroidal anti-inflammatory drugs (NSAID's).

Examples of NSAID's include sodium cromoglycate, nedocromil sodium, phosphodiesterase (PDE) inhibitors (for example, theophylline, PDE4 inhibitors or mixed PDE3/PDE4 inhibitors), leukotriene antagonists, inhibitors of leukotriene synthesis (for example montelukast), iNOS inhibitors, tryptase and elastase inhibitors, beta-2 integrin antagonists and adenosine receptor agonists or antagonists (e.g. adenosine 2a agonists), cytokine antagonists (for example chemokine antagonists, such as a CCR3 antagonist) or inhibitors of cytokine synthesis, or 5-lipoxygenase inhibitors. In one embodiment, the invention encompasses iNOS (inducible nitric oxide synthase) inhibitors for oral administration. Examples of iNOS inhibitors include those disclosed in WO1993/13055, WO1998/30537, WO2002/50021, WO1995/34534 and WO1999/62875. Examples of CCR3 inhibitors include those disclosed in WO2002/26722.

In one embodiment the invention provides the use of the present compound in combination with a phosphodiesterase 4 (PDE4) inhibitor, for example in the case of a formulation adapted for inhalation. The PDE4 inhibitor useful in this aspect of the invention may be any compound that is known to or which is discovered to act as a PDE4 inhibitor, e.g. as an inhibitor of PDE4B and/or PDE4D.

PDE4 inhibitory compounds include cis-4-cyano-4-(3-cyclopentyloxy-4-methoxyphenyl)cyclohexan-1-carboxylic acid, 2-carbomethoxy-4-cyano-4-(3-cyclopropylmethoxy-4-difluoromethoxyphenyl)cyclohexan-1-one and cis-[4-cyano-4-(3-cyclopropylmethoxy-4-difluoromethoxyphenyl)cyclohexan-1-ol]. Also, cis-4-cyano-4-[3-(cyclopenlyloxy)-4-methoxyphenyl]cyclohexane-1-carboxylic acid (also known as cilomilast) and its salts, esters, pro-drugs or physical forms, which is described in U.S. Patent 5,552,438.

Other PDE4 inhibitory compounds include AWD-12-281 (N-(3,5-dichloro-4-pyiridinyl)-1-[4-fluorophenyl)methyl]-5-hydroxy-α-oxo-1H-indol-3-acetamide) from Elbion (Hofgen, N. et al. 15th EFMC Int Symp Med Chem (Sept 6-10, Edinburgh) 1998, Abst P.98; CAS reference No. 247584020-9); a 9-benzyladenine derivative nominated NCS-613 (INSERM); D-4418 from Chiroscience and Schering-Plough; a benzodiazepine PDE4 inhibitor identified as CI-1018 (PD-168787) and attributed to Pfizer; a benzodioxole derivative disclosed by Kyowa Hakko in WO99/16766; K-34 from Kyowa Hakko; V-11294A from Napp (Landells, L.J. et al. Eur Resp J [Annu Cong Eur Resp Soc (Sept 19-23, Geneva) 1998] 1998, 12 (Suppl. 28): Abst P2393); roflumilast (3-(cyclopropylmethoxy)-N-(3,5-dichloro-4-pyridinyl)-4-(difluoromethoxy)benzamide) (see EP 0 706513 B 1 to Byk Gulden Lomberg, e.g. see Example 5 thereof); a phthalazinone (WO1999/47505) from Byk-Gulden; Pumafentrine, (-)-p-[(4aR*,10bS*)-9-ethoxy-1,2,3,4,4a,10b-hexahydro-8-methoxy-2-methylbenzo[c][1,6]naphthyridin-6-yl]-N,N-diisopropylbenzamide which is a mixed PDE3/PDE4 inhibitor which has been prepared and published on by Byk-Gulden, now Altana; arofylline under development by Almirall-Prodesfarma; VM554/UM565 from Vernalis; or T-440 (Tanabe Seiyaku; Fuji, K. et al. J Pharmacol Exp Ther, 1998, 284(1): 162), and T2585.

Further PDE4 inhibitory compounds are disclosed in the published international patent applications WO2004/024728, WO2004/056823, WO2004/103998 (e.g. Example 399 or 544 disclosed therein), WO2005/058892, WO2005/090348 WO2005/090353, and WO2005/090354, all in the name of Glaxo Group Limited.

Examples of anticholinergic agents are those compounds that act as antagonists at the muscarinic receptors, in particular those compounds which are antagonists of the M₁ or M₃ receptors, dual antagonists of the M₁/M₃ or M₂/M₃, receptors or pan-antagonists of the M₁/M₂/M₃) receptors. Exemplary compounds for administration via inhalation include ipratropium (for example, as the bromide, CAS 22254-24-6, sold under the name Atrovent), oxitropium (for example, as the bromide, CAS 30286-75-0) and tiotropium (for example, as the bromide, CAS 136310-93-5, sold under the name Spiriva). Also of interest are revatropate (for example, as the hydrobromide, CAS 262586-79-8) and LAS-34273 which is disclosed in WO2001/04118. Exemplary compounds for oral administration include pirenzepine (CAS 28797-61-7), darifenacin (CAS 133099-04-4, or CAS 133099-07-7 for the hydrobromide sold under the name Enablex), oxybutynin (CAS 5633-20-5, sold under the name Ditropan), terodiline (CAS 15793-40-5), tolterodine (CAS 124937-51-5; or CAS 124937-52-6 for the tartrate, sold under the name Detrol), otilonium (for example, as the bromide, CAS 26095-59-0, sold under the name Spasmomen), trospium chloride (CAS 10405-02-4) and solifenacin (CAS 242478-37-1, or CAS 242478-38-2 for the succinate also known as YM-905 and sold under the name Vesicare).

Additional compounds are disclosed in WO 2005/037280, WO 2005/046586 and WO 2005/104745. The present combinations include, but are not limited to:
(3-endo)-3-(2,2-di-2-thienylethenyl)-8,8-dimethyl-8-azoniabicyclo[3.2.1]octane iodide;
(3-endo)-3-(2-cyano-2,2-diphenylethyl)-8,8-dimethyl-8-azoniabicyclo[3.2.1]octane bromide;
4-[hydroxy(diphenyl)methyl]-1-{2-[(phenylmethyl)oxy]ethyl}-1-azoniabicyclo[2.2.2]octane bromide; and
(1R,5S)-(2-cyano-2,2-diphenylethyl)-8-methyl-8-{2-[(phenylmethyl)oxy]ethyl}-8-azoniabicyclo[3.2.1]octane bromide.

Other anticholinergic agents include compounds which are disclosed in US patent application 60/487981. These include for example:
(endo)-3-(2-methoxy-2,2-di-thiophen-2-yl-ethyl)-8,8-dimethyl-8-azonia-bicyclo[3.2.1]octane iodide;
3-((endo)-8-methyl-8-aza-bicyclo[3.2.1 ]oct-3-yl)-2,2-diphenyl-propionitrile;
(endo)-8-methyl-3-(2,2,2-triphenyl-ethyl)-8-aza-bicyclo[3.2.1 ]octane;
3-((endo)-8-methyl-8-aza-bicyclo[3.2.1]oct-3-yl)-2,2-diphenyl-propionamide;
3-((endo)-8-methyl-8-aza-bicyclo[3.2.1]oct-3-yl)-2,2-diphenyl-propionic acid;
(endo)-3-(2-cyano-2,2-diphenyl-ethyl)-8,8-dimethyl-8-azonia-bicyclo[3.2.1]octane iodide;
(endo)-3-(2-cyano-2,2-diphenyl-ethyl)-8,8-dimethyl-8-azonia-bicyclo[3.2.1 ]octane bromide;
3-((endo)-8-methyl-8-azabicyclo[3.2.1]oct-3-yl)-2,2-diphenyl-propan-1-ol;
N-benzyl-3-((endo)-8-methyl-8-aza-bicyclo[3.2.1]oct-3-yl)-2,2-diphenyl-propionamide;
(endo)-3-(2-carbamoyl-2,2-diphenyl-ethyl)-8,8-dimethyl-8-azonia-bicyclo[3.2.1]octane iodide;
1-benzyl-3-[3-((endo)-8-methyl-8-aza-bicyclo[3.2.1]oct-3-yl)-2,2-diphenyl-propyl]-urea;
1-ethyl-3-[3-((endo)-8-methyl-8-aza-bicyclo[3.2.1]oct-3-yl)-2,2-diphenyl-propyl]-urea;
N-[3-((endo)-8-methyl-8-aza-bicyclo[3.2.1]oct-3-yl)-2,2-diphenyl-propyl]-acetamide;
N-[3-((endo)-8-methyl-8-aza-bicyclo[3.2.1]oct-3-yl)-2,2-diphenyl-propyl]-benzamide;
3-((endo)-8-methyl-8-aza-bicyclo[3.2.1]oct-3-yl)-2,2-di-thiophen-2-yl-propionitrile;
(endo)-3-(2-cyano-2,2-di-thiophen-2-yl-ethyl)-8,8-dimethyl-8-azonia-bicyclo[3.2.1]octane iodide;
N-[3-((endo)-8-methyl-8-aza-bicyclo[3.2.1]oct 3-yl)-2,2-diphenyl-propyl]-benzenesulfonamide;
[3-((endo)-8-methyl-8-aza-bicyclo[3.2.1]oct-3-yl)-2,2-diphenyl-propyl]-urea;
N-[3-((endo)-8-methyl-8-aza-bicyclo[3.2.1]oct-3-yl)-2,2-diphenyl-propyl]-methanesulfonamide; and/or
(endo)-3-{2,2-diphenyl-3-[(1-phenyl-methanoyl)-amino]-propyl}-8,8-dimethyl-8-azonia-bicyclo[3.2.1]octane bromide.

Further compounds include:
(endo)-3-(2-methoxy-2,2-di-thiophen-2-yl-ethyl)-8,8-dimethyl-8-azonia-bicyclo[3.2.1]octane iodide;
(endo)-3-(2-cyano-2,2-diphenyl-ethyl)-8,8-dimethyl-8-azonia-bicyclo[3.2.1]octane iodide;
(endo)-3-(2-cyano-2,2-diphenyl-ethyl)-8,8-dimethyl-8-azonia-bicyclo[3.2.1]octane bromide;
(endo)-3-(2-carbamoyl-2,2-diphenyl-ethyl)-8,8-dimethyl-8-azonia-bicyclo[3.2.1]octane iodide;
(endo)-3-(2-cyano-2,2-di-thiophen-2-yl-ethyl)-8,8-dimethyl-8-azonia-bicyclo[3.2.1]octane iodide; and/or
(endo)-3- {2,2-diphenyl-3-[(1-phenyl-methanoyl)-amino]-propyl}-8,8-dimethyl-8-azonia-bicyclo[3.2.1]octane bromide.

In one embodiment the invention provides a combination comprising the present compound together with an H1 antagonist. Examples of H1 antagonists include, without limitation, amelexanox, astemizole, azatadine, azelastine, acrivastine, brompheniramine, cetirizine, levocetirizine, efletirizine, chlorpheniramine, clemastine, cyclizine, carebastine, cyproheptadine, carbinoxamine, descarboethoxyloratadine, doxylamine, dimethindene, ebastine, epinastine, efletirizine, fexofenadine, hydroxyzine, ketotifen, loratadine, levocabastine, mizolastine, mequitazine, mianserin, noberastine, meclizine, norastemizole, olopatadine, picumast, pyrilamine, promethazine, terfenadine, tripelennamine, temelastine, trimeprazine and triprolidine, particularly cetirizine, levocetirizine, efletirizine and fexofenadine. In a further embodiment the invention provides a combination comprising the present compound together with an H3 antagonist (and/or inverse agonist). Examples of H3 antagonists include, for example, those compounds disclosed in WO2004/035556 and in WO2006/045416. Other histamine receptor antagonists which may be used in combination with the compounds of the present invention include antagonists (and/or inverse agonists) of the H4 receptor, for example, the compounds disclosed in Jablonowski et al., J. Med. Chem. 46:3957-3960 (2003).

In one embodiment, the invention provides a combination comprising the present compound together with a CCR5 receptor antagonist, such as 4,4-difluoro-N-((1S)-3-{3-[3-methyl-5-(1-methylethyl)-4H-1,2,4-triazol-4-yl]-8-azabicyclo[3.2.1.]oct-8-yl}-1-phenylpropyl)cyclohexanecarboxamide:

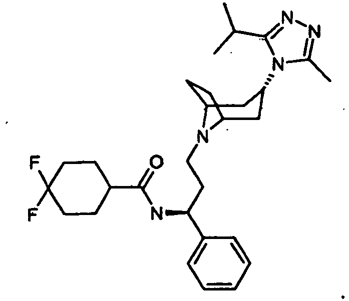

In one embodiment, the invention provides a combination comprising the present compound together with a CXCR3 receptor antagonist such as N-((1R)-1-{3-[4-(ethyloxy)phenyl]-4-oxo-3,4-dihydropyrido[2,3-d]pyrimidin-2-yl}ethyl)-N-(3-pyridinylmethyl)-2-{4-[(trifluoromethyl)oxy]phenyl}acetamide:

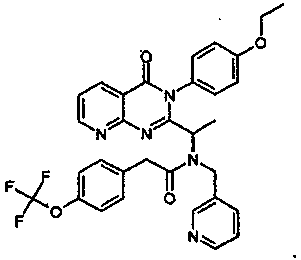

The invention thus provides, in a further aspect, a combination comprising the present compound together with a PDE4 inhibitor.

The invention thus provides, in a Further aspect, a combination comprising the present compound together with a β₂-adrenoreceptor agonist.

The invention thus provides, in a further aspect, a combination comprising the present compound together with a corticosteroid.

The invention thus provides, in a further aspect, a combination comprising the present compound together with a non-steroidal GR agonist.

The invention thus provides, in a further aspect, a combination comprising the present compound together with an anticholinergic agent.

The invention thus provides, in a further aspect, a combination comprising the present compound together with an antihistamine.

The invention thus provides, in a further aspect, a combination comprising the present compound together with a PDE4 inhibitor and a β₂-adrenoreceptor agonist.

The invention thus provides, in a Further aspect, a combination comprising the present compound together with an anticholinergic and a PDE-4 inhibitor.

The combinations referred to above may conveniently be presented for use in the form of a pharmaceutical formulation and thus pharmaceutical formulations comprising a combination as defined above together with u pharmaceutically acceptable diluent or carrier represent a further aspect of the invention.

The individual compounds of such combinations may be administered either sequentially or simultaneously in separate or combined pharmaceutical formulations. In one embodiment, the individual compounds will be administered simultaneously in a combined pharmaceutical formulation. Appropriate doses of known therapeutic agents will readily be appreciated by those skilled in the art.

The invention thus provides, in a further aspect, a pharmaceutical composition comprising a combination of the present compound together with another therapeutically active agent.

The invention thus provides, in a further aspect, a pharmaceutical composition comprising a combination of the present compound together with a PDE4 inhibitor.

The invention thus provides, in a further aspect, a pharmaceutical composition comprising a combination of the present compound together with a β₂-adrenoreceptor agonist.

The invention thus provides, in a further aspect, a pharmaceutical composition comprising a combination of the present compound together with a corticosteroid.

The invention thus provides, in a further aspect, a pharmaceutical composition comprising a combination of the present compound together with a non-steroidal GR agonist.

The invention thus provides, in a further aspect, a pharmaceutical composition comprising a combination of the present compound together with an anticholinergic.

The invention thus provides, in a further aspect, a pharmaceutical composition comprising a combination of the present compound together with an antihistamine.

The invention thus provides, in a further aspect, a pharmaceutical composition comprising a combination of the present compound together with CXCR3 receptor antagonist.

The invention thus provides, in a further aspect, a pharmaceutical combination of the invention together with a CCR5 receptor antagonist.

The invention will now be described by reference to the following biological examples which are merely illustrative and are not to be construed as a limitation of the scope of the present invention.

### BIOLOGICAL EXAMPLES

The IL-8, and GRO-α chemokine inhibitory effects of compounds of the present invention are determined by the following *in vitro* assay:

### Receptor Binding Assay:

[¹²⁵I] IL-8 (human recombinant) is obtained from GE Healthcare, with specific activity 2000 Ci/mmol. All other chemicals are of analytical grade. High levels of recombinant human CXCR1 (IL-8 type α) and CXCR2 (IL-8 type β) receptors are individually expressed in non-adherent Chinese Hamster Ovary (CHO) cells as described previously (Holmes, et al., Science,1991, 253, 1278). The membranes are prepared according to a previously described protocol, Haour, et al., J. Biol. Chem., 249 pp 2195-2205 (1974)), except that the homogenization buffer is modified to 40mM Tris-HCL (pH 7.5), 1mM MgS0₄, 0.5 mM EGTA (ethylene- glycol-bis(2-aminoethylether)- N,N,N',N' tetra-acetic acid), 1mM PMSF (α-toluenesulphonyl fluoride), 2.5 mg/L leupeptin and 0.1 mg/ml aprotinin. Cells are homogenized and centrifuged at 2,000 rpm for 10 min. The supernatant is centrifuged at 100,000 x g for 1 hour. The supernatant is discarded and membranes stored at -80°C. Membrane protein concentration is determined using BioRad reagent according to manufactures protocol using bovine serum albumin (BSA) as a standard.

All IL-8 binding is conducted using Scintillation Proximity Assays (SPA) using wheatgerm agglutinin beads in a 96-well plate format. Membranes CHO-CXCR1 or CHO-CXCR2 are preincubated with the beads in the binding buffer for 30 min. for 4°C Buffer contains 20 mM Bis-Trispropane buffer, pH 8.0, containing 1 mM MgSO₄,0.1 mM EDTA and 25 mM NaCl. Compounds are diluted in DMSO at 20X the final dilution (final compound concentration between 1 nM and 30 uM and final DMSO concentration of 5%). Assay is performed in 96-well plates (optiplate 96, Packard) at room temperature, in 0.1 ml binding buffer with membranes and 0.04% CHAPS (3-[(3-cholamidopropyl) dimethylammonio]-1-propanesulfonate), 0.0025% BSA and 0.23 nM [¹²⁵I] IL-8. Plates are shaken on a platform for 1 hour, at the end of incubation the plates were spun at 2,000 rpm for 5 min and counted in a Top Count counter The recombinant IL-8 Rα, CXCR1 or Type I, receptor is also referred to herein as the non-permissive receptor and the recombinant IL-8 Rβ, CXCR2 or Type II, receptor is referred to as the permissive receptor.

A compound is considered active in this assay if it exhibits an IC₅₀ value of < 30 uM. The present compound is expected to test active at an IC₅₀ value of about 13 nM in the present assay.

### Chemotaxis Assay:

A neutrophil chemotaxis assay is performed. Primary human neutrophils are isolated from peripheral whole blood using percoll discontinuous gradient centrifugation, dextran sedimentation and hypotonic lysis. The chemoattractants IL-8 (CXCL8) or GRO-α (CXCL1) are placed in the bottom chamber of a 96 multi-well chamber (ChemoTx System, Neuro Probe, Gaithersburg, MD). The agonist concentration used is an EC80 concentration. The two chambers are separated by a 5 um polycarbonate membrane. A compound tested is preincubated with the cells prior to placement on the top of the filter. Chemotaxis is allowed to proceed for 45 minutes in a humidified incubator at 37°C with 5% CO₂. At the end of the incubation period, the membrane is removed and the migrated cells in the bottom chamber are transferred to a 96-well plate. These cells are measured using a luminescent cell viability assay (Celltiter-Glo, Promega, Madison, WI). Each sample is tested in duplicate and each compound repeated at least three times. Positive control cells are cells without compound added and represent the maximum chemotactic response. The negative control (unstimulated) is with no chemokine added to the bottom chamber. The difference between the positive control and the negative control represents the chemotactic activity of the cells.

A compound is considered active if it exhibits an IC₅₀ value of <5 uM.

### CD11b Human Whole Blood assay:

A compound is tested for its ability to inhibit the GROα-induced expression of the integrin CD11b on neutrophils in human whole blood.

Blood is drawn (9 ml) using a butterfly line and a 10 ml syringe containing 0.2 ml of working Sodium Heparin. The blood is kept at 37°C until placed on ice in step 5 below. Compound stock solutions are then diluted to 12 times the maximum final concentration, 120 uM. Half Log serial dilutions are then performed in vehicle. Ten microliters of the compound dilutions or vehicle are then added to the appropriate 12x75 polypropylene tubes. One hundred microliters of whole blood is added per tube and incubated for 10 minutes, in a 37°C water bath with initial (gentle) agitation and again at 5 minutes. The GROα stock is diluted 1:166.66 in 0.1% BSA-DPBS to "12x" concentration of 120 nM and 10 ul of the GROα dilution or 0.1%BSA-DPBS is added to the appropriate tubes so that the final GROα concentration equals 10 nM. The tubes are incubated for 10 min at 37°C with gentle hand agitation and again at 5 minutes. Samples are then placed on ice and 250 ul of ice cold CellFix working dilution is added followed by a one minute incubation on ice. 1.5 ml Eppendorf tubes are readied during GROα incubation by adding the appropriate antibodies. Every tube receives 10 ul of CD11b-FITC and 5 ul of CD16-PE, except for the isotype control which receives 10 ul of IgG2a-FITC instead of CD11b. Addition of 50 ul of the fixed blood from each tube is added to the appropriate Eppendorf tube. Samples are allowed to then incubate for 20 min at 4°C in the dark. Addition of the blood/antibody mixtures to 500 ul of cold DPBS are added to the appropriately labeled 12×75 polystyrene tube. The resulting mixture is kept on ice. LDS stock (10 ul) is added and the mixture incubated for 10 min at 4°C. before flow analysis. Samples are kept in a darkened environment. The LDS addition is staggered as the samples are collected on the flow cytometer so that all samples are run ~10-20 minutes post-LDS addition.

Medium flow rate is used for flow collection and FL3 threshold increased to eliminate red blood cells from analysis using the LDS signal. The color compensation is properly set using unlabeled samples and one-color samples to subtract LDS spill into PE and the PE spill into FITC and FITC into PE. For the BD LSR cytometer, LDS=FL3, PE=FL2, FTTC=FL1. A minimum of 2000-3000 events that satisfy the granulocyte gate by SSC vs. FSC and are CD16 positive by the FL2 signal are collected.

A compound is considered active in this assay if it exhibits an IC₅₀ value of <5uM.

### Calcium Mobilization in CHO-K1 cells stably expressing CXCR2 and Gα16:

CHO-Klcells stably expressing CXCR2 and Gα16 are grown to 80% confluency in DMEM/F12 (HAM's)1:1, w/ 10% FCS (heat inactivated), w/ 2 mM L-glutamine, w/0.4 mg/ml G418 while maintained at 37°C in a 5% CO₂ incubator. Twenty four hours previous to assay, cells are harvested and plated, 40,000 cells per well, in a 96 well, black wall, clear bottom plate (Packard View) and returned to CO₂ incubator. On the day of assay, compounds are serially diluted in 100% DMSO to 300X the desired assay concentration. Growth media is aspirated off cells and replaced with 100 ul of load media (EMEM with Earl"s salts w/L-Glutamine, 0.1% BSA, (BovuminarCohen Fraction V from Seriologicals Corp.), 4 uM Fluo-4-acetoxymethyl ester fluorescent indicator dye (Fluo-4 AM, from Molecular Probes), and 2.5 mM probenecid) and incubated for 1 hour at 37°C. in CO₂ incubator. Load media is aspirated and replaced with 100 uL of EMEM with Earl"s salts w/L-Glutamine, 0.1% gelatin, and 2.5 mM probenecid and incubated for an additional 10 min. Serially diluted compound (3 ul) in DMSO at 300X is transferred to a 96 well plate containing 297 micro liters of KRH (120 mM NaCl, 4.6mM KCl, 1.03 mM KH₂PO₄, 25 mM NaHCO₃, 1.0mM CaCl₂, 1.1mM MgCl₂, 11mM Glucose, 20 mM HEPES (pH 7.4)) w/ 2.5 mM probenecid and 0.1% gelatin (compound now at 3X). Media is aspirated off cells, and cells washed 3 times with KRH w/ 2.5 mM probenecid, w/ 0.1% gelatin. KRH (100 ul) w/ 2.5 mM probenecid with 0.1% gelatin is added to wells then 50 ul of 3X compound in KRH w/ 2.5 mM probenecid and 0.1% gelatin is added to wells (compound now at 1X) and incubated at 37°C in CO₂ incubator for 10 min. Plates are placed onto FLIPR (Fluorometric Imaging Plate Reader, Molecular Devices, Sunnyvale CA) for analysis as described previously (Sarau et al., 1999). The percent of maximal human IL-8 induced Ca²⁺ mobilization induced by 1.0 nM IL-8, an EC₈₀ conc. for CXCR2, is determined for each concentration of compound and the IC₅₀ calculated as the concentration of test compound that inhibits 50% of the maximal response induced by 1.0 nM IL-8. A compound is considered active in this assay if it exhibits an IC₅₀ value of <10uM.

### Netitrophil CD11b Stimulation Following Oral Dosing to Rats

Lewis rats (250-300 gm) were dosed orally with the present compound or vehicle and one hour later they were euthanized by CO₂ asphyxiation. Rat whole blood, 3 ml, was drawn by cardiac puncture in a syringe containing 100 µl of 0.25 M EDTA (GIBCO, Grand Island, NY). Rat CXCL2 (PeproTech, Rocky Hill, NJ) stock was made by reconstitution in Kreb's/0.1 % BSA (KBSA) at 10 µM. The stock was diluted to "11 x" the maximum concentration used in DPBS (GIBCO) and serially diluted in KBSA/DPBS vehicle. Ten µl of appropriate concentration of the present compound (1.2-100 nM) or vehicle was added to 12x75 mm polypropylene tubes followed by 100 µl of whole blood. The tubes were incubated for 30 minutes in a 37°C bath, with gentle hand agitation every 10 minutes. The samples were then placed on ice for 10 minutes followed by addition of 10 µl of anti-rat-CD11b-FlTC or FITC-labeled mouse IgG2a isotype control (both Antigenix America, Huntington Station, NY) and incubated for 30 minutes on ice. FACS Lysing Solution (Becton Dickinson, San Jose, CA), 1 ml of 1X, was added with immediate vigorous vortexing, followed by additional vortex after the solution was added to the last sample. Samples were incubated for 10 minutes at room temperature and the leukocytes were pelleted at ~300 x g and washed with DPBS. Cells were resuspended in 650 µl of 1% paraformaldahyde. The FACS Lyse solution does not completely lyse rat red blood cells. Therefore, for flow cytometric analysis, 3.5 µl of a 1.67 mg/ml ethanol solution (supersaturated; clarified by centrifugation) of LDS-751 (Exciton, Dayton, OH) was added to each sample within 1-2 minutes of flow analysis to gate out any remaining red blood cells. Sample data was collected using CellQuest Software and an LSR flow cytometer (Becton-Dickinson), on Low flow rate setting, by increasing the FL3 threshold to eliminate the LDS-751 negative red blood cells and then gating on the neutrophil population in the side scatter versus forward scatter plot. FL1 (green FITC fluorescence, directly relating to CD 11b content) of this population was then measured as mean channel fluorescence by analysis with CellQuest software.

In the present assay, the present compound tested active for inhibition of whole blood neutrophil CD11b expression following oral dosing to Lewis rats at 10 mg/kg. The present compound significantly shifted the EC50 of the CXCL2 concentration response curve from 4.7 nM (1.9 - 7.4; 95% C.I.) in vehicle-treated rats to 12.3 nM (10.0 - 14.7), (p<0.001, n=6 per group).

The above description fully discloses the invention including preferred embodiments thereof. Modifications and improvements of the embodiments specifically disclosed herein are within the scope of the following claims. Without further elaboration, it is believed that one skilled in the art can, using the preceding description, utilize the present invention to its fullest extent. Therefore the Examples herein are to be construed as merely illustrative and not a limitation of the scope of the present invention in any way. The embodiments of the invention in which an exclusive property or privilege is claimed are defined as follows.

## Claims

1. A compound welch is N-[4-chloro-2-hydroxy-3-(pipprarine-1-sulfonyl)phenyl]-N'-(2-chloro-3-fluoro phenyl)urea p-toluenesulfonate.

2. A pharmaceulical composition comprising N-[4-chloro-2-hydroxy-3-(piperazine-1-sutfonyl)phenyl]-N'-(2-chloro-3-(fluorophenyl)urea p-toluenesulfonate and a pharmaceutically acceptable carrier or diluent

3. The use of N-[4-chloro-2-hydroxy-3-(piperazine·1-sultonyl)phenyl]-N'-(2-chloro-3-fluorophenyl)urea p-toluonesuffonate in the manufacture of a médicament for the treatment of a chemokine mediated disease.

4. The use of N-[4-chloro-2-hydroxy-3-(piperazine-1-sulfonyl)phenyl]-N'-(2-chloro-3-fluorophenyl)urea p-toluenesulfonate in the manufacture of a medicament according to claim 3 for the treatment of asthma, chronic obstructive pulmonary disease or adult respiratory distress syndrome.

5. The use of N-[4-chloro-2-hydroxy-3-(piperazine-1-sulfonyl)phenyl]-N'-(2-chloro-3-fluorophenyl)urea p-toluenesulfonate in the manufacture of a medicament according to claim 4 for the treatment of chronic obstructive pulmonary disease.

6. A pharmaceutical composition comprising N-[4-chloro-2-hydroxy-3-(piperazine-1-sulfonyl)phenyl]-N'-(2-chloro-3-fluofophenyl)urea p-toluenesulfonate and one or more additional therapeutic ingredlents.

7. The composillon according to claim 6 wherein the additional therapeutic ingredient is a CXCR3 receptor antagonist or a CCR5 receptor antagonist.

8. A method of preparing N-[4-chloro-2-hydroxy-3-(-piperazine-1-sulfonyl)-phenyl]N'-(2-chloro-3-fluorophenyl)urea p-toluenesulfonate comprlsing the steps of:
a) combining acetonitrile and p-toluenesulfonic acid monohydrate;
b) adding the product of step a) to 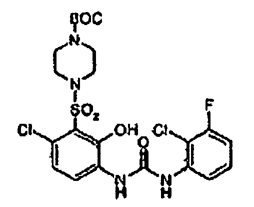 dissolved in tetrahydrofuran: and
c) treating the product obtained in step b) with acetonitrile, and deprotecting the product of step b.

9. A compound which is N-[4-chloro-2-hydroxy-3-(piperazine-1-sulfonyl)phenyl]-N'-(2-chloro-3-fluorophenyl)urea p-toluenesulfonate for use as an active therapeutic substance.

10. A compound which is N-[4-chloro-2-hydroxy-3-(piperazine-1-sulfonyl)phenyl]-N'-(2-chloro-3-fluorophenyl)urea p-toluenesulfonate for use in the treatment of a chemokine mediated disease.

11. A compound which is N-[4-chloro-2-hydroxy-3-(piperazine-1-sulfonyl)phenyl]-N'-(2-chloro-3-fluorophenyl)urea p-toluenesulfonate for use according to claim 10 in the treatment of asthma, chronic obstructive pulmonary disease or adult respiratory distress syndrome.

12. A compound which is N-[4-chloro-2-hydroxy-3-(piperazine-9-sulfonyl)phenyl]-N'-(2-chloro-3-fluorophenyl)urea p-toluenesulfonate for use according to claim 11 in the treatment of chronic obstructive pulmonary disease.

## Patentansprüche

1. Verbindung, die N-[4-Chlor-2-hydroxy-3-(piperazin-1-sulfonyl)phenyl]-N'-(2-chlor-3-fluorphenyl)harnstoff-p-toluolsulfonat ist.

2. Pharmazeutische Zusammensetzung, umfassend N-[4-Chlor-2-hydroxy-3-(piperazin-1-sulfonyl)phenyl]-N'-(2-chlor-3-fluorphenyl)harnstoff-p-toluolsulfonat und ein(en) pharmazeutisch annehmbare(n/s) Träger oder Verdünnungsmittel.

3. Verwendung von N-[4-Chlor-2-hydroxy-3-(piperazin-1-sulfonyl)phenyl]-N'-(2-chlor-3-fluorphenyl)harnstoff-p-toluolsulfonat bei der Herstellung eines Medikaments zur Behandlung einer durch Chemokin vermittelten Krankheit.

4. Verwendung von N-[4-Chlor-2-hydroxy-3-(piperazin-1-sulfonyl)phenyl]-N'-(2-chlor-3-fluorphenyl)harnstoff-p-toluolsulfonat bei der Herstellung eines Medikaments gemäss Anspruch 3 zur Behandlung von Asthma, chronisch obstruktiver Lungenerkrankung oder Atemnotsyndrom bei Erwachsenen.

5. Verwendung von N-[4-Chlor-2-hydroxy-3-(piperazin-1-sulfonyl)phenyl]-N'-(2-chlor-3-fluorphenyl)harnstoff-p-toluolsulfonat bei der Herstellung eines Medikaments gemäss Anspruch 4 zur Behandlung von chronisch obstruktiver Lungenerkrankung.

6. Pharmazeutische Zusammensetzung, umfassend N-[4-Chlor-2-hydroxy-3-(piperazin-1-sulfonyl)phenyl]-N'-(2-chlor-3-fluorphenyl)harnstoff-p-toluolsulfonat und einen oder mehrere weitere(n) therapeutische(n) Bestandteil (e).

7. Zusammensetzung gemäss Anspruch 6, wobei der weitere therapeutische Bestandteil ein CXCR3-Rezeptor-Antagonist oder ein CCRS-Rezeptor-Antagonist ist.

8. Verfahren zur Herstellung von N-[4-Chlor-2-hydroxy-3-(piperazin-1-sulfonyl)phenyl]-N'-(2-chlor-3-fluorphenyl)harnstoff-p-toluolsulfonat, umfassend die Schritte:
(a) Kombinieren von Acetonitril und p-Toluolsulfonsäuremonohydrat;
(b) Zugeben des Produkts aus Schritt (a) zu 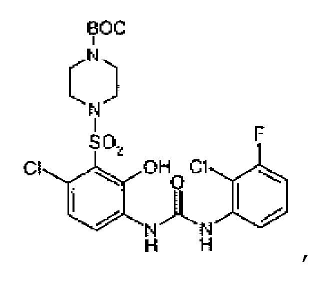 das in Tetrahydrofuran aufgelöst ist; und
(c) Behandeln des in Schritt (b) erhaltenen Produkts mit Acetonitril und Entschützen des Produkts aus Schritt (b).

9. Verbindung, die N-[4-Chlor-2-hydroxy-3-(piperazin-1-sulfonyl)phenyl]-N'-(2-chlor-3-fluorphenyl)harnstoff-p-toluolsulfonat ist, zur Verwendung als therapeutisch wirksame Substanz.

10. Verbindung, die N-[4-Chlor-2-hydroxy-3-(piperazin-1-sulfonyl)phenyl]-N'-(2-chlor-3-fluorphenyl)harnstoff-p-toluolsulfonat ist, zur Verwendung bei der Behandlung einer durch Chemokin vermittelten Krankheit.

11. Verbindung, die N-[4-Chlor-2-hydroxy-3-(piperazin-1-sulfonyl)phenyl]-N'-(2-chlor-3-fluorphenyl)harnstoff-p-toluolsulfonat ist, zur Verwendung gemäss Anspruch 10 bei der Behandlung von Asthma, chronisch obstruktiver Lungenerkrankung oder Atemnotsyndrom bei Erwachsenen.

12. Verbindung, die N-[4-Chlor-2-hydroxy-3-(piperazin-1-sulfonyl)phenyl]-N'-(2-chlor-3-fluorphenyl)harnstoff-p-toluolsulfonat ist, zur Verwendung gemäss Anspruch 11 bei der Behandlung von chronisch obstruktiver Lungenerkrankung.

## Revendications

1. Composé qui est le p-toluènesulfonate de N-[4-chloro-2-hydroxy-3-(pipérazine-1-sulfonyl)phényl]-N'-(2-chloro-3-fluorophényl)urée.

2. Composition pharmaceutique comprenant du p-toluènesulfonate de N-[4-chloro-2-hydroxy-3-(pipérazine-1-sulfonyl)phényl]-N'-(2-chloro-3-fluoro-phényl)urée et un support ou un diluant pharmaceutiquement acceptable.

3. Utilisation de p-toluènesulfonate de N-[4-chloro-2-hydroxy-3-(pipérazine-1-sulfonyl)phényl]-N'-(2-chloro-3-fluorophényl)urée dans la fabrication d'un médicament destiné au traitement d'une maladie médiée par une chimiokine.

4. Utilisation de p-toluènesulfonate de N-[4-chloro-2-hydroxy-3-(pipérazine-1-sulfonyl)phényl]-N'-(2-chloro-3-fluorophényl)urée dans la fabrication d'un médicament selon la revendication 3 pour le traitement de l'asthme, d'une bronchopneumopathie chronique obstructive ou du syndrome de la détresse respiratoire de l'adulte.

5. Utilisation de p-toluènesulfonate de N-[4-chloro-2-hydroxy-3-(pipérazine-1-sulfonyl)phényl]-N'-(2-chloro-3-fluorophényl)urée dans la fabrication d'un médicament selon la revendication 4 pour le traitement d'une bronchopneumopathie chronique obstructive.

6. Composition pharmaceutique comprenant du p-toluènesulfonate de N-[4-chloro-2-hydroxy-3-(pipérazine-1-sulfonyl)phényl]-N'-(2-chloro-3-fluoro-phényl)urée et un ou plusieurs composants thérapeutiques supplémentaires.

7. Composition selon la revendication 6, dans laquelle le composant thérapeutique supplémentaire est un antagoniste du récepteur CXCR3 ou un antagoniste du récepteur CCR5.

8. Procédé de préparation de p-toluènesulfonate de N-[4-chloro-2-hydroxy-3-(pipérazine-1-sulfonyl)phényl]-N'-(2-chloro-3-fluorophényl)urée comprenant les étapes suivantes :
a) la combinaison d'acétonitrile et d'acide p-toluène sulfonique monohydraté ;
b) l'addition du produit de l'étape a) à 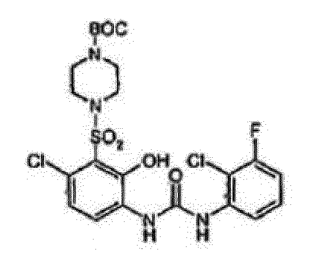 dissous dans du tétrahydrofurane ; et
c) le traitement du produit obtenu dans l'étape b) avec de l'acétonitrile, et la déprotection du produit de l'étape b).

9. Composé qui est le p-toluènesulfonate de N-[4-chloro-2-hydroxy-3-(pipérazine-1-sulfonyl)phényl]-N'-(2-chloro-3-fluorophényl)urée pour une utilisation en tant que substance thérapeutique active.

10. Composé qui est le p-toluènesulfonate de N-[4-chloro-2-hydroxy-3-(pipérazine-1-sulfonyl)phényl]-N'-(2-chloro-3-fluorophényl)urée pour une utilisation dans le traitement d'une maladie médiée par une chimiokine.

11. Composé qui est le p-toluènesulfonate de N-[4-chloro-2-hydroxy-3-(pipérazine-1-sulfonyl)phényl]-N'-(2-chloro-3-fluorophényl)urée pour une utilisation selon la revendication 10 dans le traitement de l'asthme, d'une bronchopneumopathie chronique obstructive ou du syndrome de la détresse respiratoire de l'adulte.

12. Composé qui est le p-toluènesulfonate de N-[4-chloro-2-hydroxy-3-(pipérazine-1-sulfonyl)phényl]-N'-(2-chloro-3-fluorophényl)urée pour une utilisation selon la revendication 11 dans le traitement d'une bronchopneumopathie chronique obstructive.
